# EUROPEAN PATENT APPLICATION

(11) **EP 3 770 251 A1**
(43) Date of publication of application: **27.01.2021**
(21) Application number: 19770939.7
(22) Date of filing: 22.03.2019
(51) Int. Cl.: C12N 5/0783

(54) **METHOD FOR PRODUCING NATURAL KILLER CELLS**

(30) Priority: 23.03.2018 KR 20180033828; 20.03.2019 KR 20190031875
(71) Applicant: Green Cross Lab Cell Corporation, Yongin-si, Gyeonggi-do 16924 (KR)
(72) Inventor: HWANG, Yu-Kyeong, Seoul 06359 (KR); PAIK, Sang Hoon, Yongin-si Gyeonggi-do 16923 (KR); HAN, Seungryel, Hwaseong-si Gyeonggi-do 18488 (KR); LEE, Sanghyun, Yongin-si Gyeonggi-do 16827 (KR); LAM, Hyeongjin, Yongin-si Gyeonggi-do 16840 (KR); KIM, Juyoung, Yongin-si Gyeonggi-do 16833 (KR); HAN, Mu Ri, Yongin-si Gyeonggi-do 16924 (KR); NOH, Dong Il, Yongin-si Gyeonggi-do 16924 (KR)
(74) Representative: Hughes, Sean David
(86) International application number: PCT/KR2019/003341
(87) International publication number: WO 2019/182392

(57) **Abstract**

The present disclosure relates to a method for producing natural killer (NK) cells. More specifically, the present disclosure relates to a method for producing NK cells, characterized in that peripheral blood mononuclear cells from which CD3-positive cells are removed are proliferated together with feeder cells, and the peripheral blood mononuclear cells are re-stimulated with feeder cells at the time of reaching a specific accumulated population doubling level. The present disclosure also relates to a method for producing NK cells, characterized in that NK cells are cultured under appropriate culture conditions by using a bioreactor. The production method according to the present disclosure has an advantage that NK cells having a high cell-killing ability and cell survival rate can be produced with high purity and at high efficiency in a short period of time by a clinically friendly method as compared with existing methods, thereby increasing the productivity of an NK cell therapy agent.

## Description

### [Technical Field]

The present disclosure relates to a method for producing natural killer (NK) cells, more specifically to a method for producing NK cells, characterized in that peripheral blood mononuclear cells from which CD3-positive cells are removed are proliferated together with feeder cells and the peripheral blood mononuclear cells are re-stimulated with feeder cells at the time of reaching a specific accumulated population doubling level.

The present disclosure also relates to a method for producing NK cells, characterized in that NK cells are cultured under appropriate culture conditions by using a bioreactor.

### [Background Art]

Natural killer (NK) cells are lymphoid cells which account for about 10% of blood cells and play important roles in immune responses. NK cells have many functions. In particular, they serve to remove abnormal cells that have undergone or are undergoing tumorization since they have the ability to kill cancer cells, cells infected with pathogens that have invaded from outside, etc.

Most NK cells are normally present in the body in an inactivated state. But, in order to use the NK cells for therapeutic purposes, the NK cells need to be activated. Thus, studies on activation of NK cells from normal blood or from the blood of patients with inactivated NK cells have been conducted actively.

The high cytotoxicity of NK cells, achieved by activating NK cells ex *vivo,* demonstrated the possibility of NK cells for use in immune cell therapy. It was reported that NK cells activated ex *vivo* have therapeutic effects on various cancers, particularly blood cancers such as leukemia, when administered to patients after allogeneic bone marrow transplantation (Blood Cells Molecules & Disease, 33: p261-266, 2004).

Meanwhile, despite the possibility of NK cells as therapeutic agents, the number of NK cells present *in vivo* is not large, and thus a technology of producing large amounts of NK cells while maintaining efficiency sufficient for therapeutic purposes is required. However, NK cells are not sufficiently cultured and proliferated in large quantities *in vitro.* Thus, a technology for culturing and expanding NK cells at useful levels has received attention and many studies have been conducted thereon. But, the study results are still not clinically applicable.

There have been studies on the culture of NK cells not only using IL-2, which has been used in T cell proliferation/activation, but also IL-15 (J. Immunol., 167(6): p3129-3138, 2001; Blood, 106(1): p158-166, 2005, Korean Patent Publication No. 2009-0121694), LPS (J. Immunol., 165(1): p139-147, 2000) and OKT-3 antibody (Experimental Hematol., 29(1): p104-113, 2001) which stimulates CD3. However, such studies have merely found a modification of the use of IL-2 and a new proliferator, but did not suggest an epochal method for proliferation. It is generally known that, when NK cells are cultured using IL-2 or other cytokines or chemicals, the number of NK cells is increased only by about 3-10 times the initial number of the NK cells.

Some researchers reported that NK cells were expanded using tumor cell lines as feeder cells. It was reported that the use of the leukemia cell line CTV-1 as feeder cells showed little improvement in proliferation (J. Immunol., 178(1): p85-94, 2007) and that culture using EBV-LCL for 21 days increased the cell number by an average of about 490 fold (Cytotherapy, 11(3): p341-355, 2009). Also, co-culture of NK cells for 3 weeks using artificial antigen-presenting cells (APC) obtained by expressing 4-1BBL and membrane-bound IL-15 in K562 cells increased the NK cell number by about 227 fold. Recently, it was reported that co-culture of NK cells for 3 weeks in K562 cells transfected with MICA, 4-1BBL and IL-15 increased the NK cell number by an average of 350 fold (Tissue Antigens, 76(6): p467-475, 2010), and there was a report that, when NK cells were cultured for 2 weeks using K562 cells transfected with membrane-bound IL-21 while they were re-stimulated at 7-day intervals, the cell number was increased by an average of 21,000 fold. However, these results were obtained using the methods unsuitable for guaranteeing safety which is important for clinical application, because cancer cells were used to proliferate the NK cells. In addition, specific cancer cells, not normal cells, are used as feeder cells, there is a limitation that the resulting NK cells have priming specificity for the specific cancer cells.

Cells obtained by selective enrichment of NK cells from peripheral blood leukocytes (PBL) without isolation of NK cells have low cell-killing ability compared to pure NK cells, and contain not only NK cells but also T cells that recognize self and non-self cells by autologous MHC molecules. Thus, the use of the cells is limited to autologous transplantation, as long as T cells are not removed.

Recently, there have been developed a method of isolating NK cells and expanding the isolated NK cells by applying suitable stimulation using feeder cells, and a method of selectively expanding NK cells using whole PBLs or peripheral blood mononuclear cells (PBMC). In addition, there was reported a method for culturing NK cells, which includes a process of culturing NK cells using a medium containing anti-CD3 antibody and interleukin protein in the presence of peripheral blood leukocytes (Korean Patent Publication No. 10-2010-0011586).

A general expansion process for allogeneic application of NK cells starts with two sequential steps of magnetic depletion of CD3+ T cells and enrichment of CD56+ NK cells. In order to stimulate NK cell proliferation, irradiated feeder cells such as PBMCs (Cytotherapy 12: 750-763, 2010) or Epstein-Barr virus-transformed lymphoblastoid cell lines (EBV-LCLs) are often used. The irradiated feeder cells stimulate NK cells through both humoral factors and direct cell-to-cell contact (Blood 80: 2221-2229, 1992).

The inventors of the present disclosure have prepared, for large-scale proliferation and activation of NK cells, a highly pure population of CD3-CD16+CD56+ NK cells by, after a single step of magnetic depletion of CD3+ T cells, stimulating T cell-removed PBMCs and proliferating them repeatedly together with inactivated feeder cells in the presence of OKT3 and IL-2 (Korean Patent Registration No. 1,644,984). However, the method is limited in obtaining high-purity NK cells in large quantities.

Therefore, the inventors of the present disclosure have made consistent efforts to develop a method for preparing high-purity NK cells having high killing ability in large quantities. As a result, they have identified that the growth of NK cells is enhanced and cell recovery rate is improved when peripheral blood mononuclear cells from which CD3-positive cells are removed are stimulated by culturing stationarily together with feeder cells, re-stimulated with feeder cells at the time when the accumulated population doubling level (aPDL) of the peripheral blood mononuclear cells reached 2-6 and then suspension-cultured, and have completed the present disclosure.

In addition, the inventors of the present disclosure have first identified that culturing of NK cells under appropriate culture conditions using a bioreactor which allows control of the dissolved oxygen concentration, dissolved carbon dioxide concentration, pH and temperature in a culture medium, adequate agitation, continuous supply of the medium, etc. remarkably improves the amount of the NK cells obtained and the killing ability of the prepared NK cell, and have completed the present disclosure.

### [Disclosure]

### [Technical Problem]

The present disclosure is directed to providing a method for producing NK cells, which allows remarkably enhanced growth of NK cells and significant improvement of NK cell recovery rate by re-stimulating mononuclear cells in a cell culture with feeder cells at the time of reaching a specific accumulated population doubling level.

The present disclosure is also directed to providing a method for producing NK cells, characterized in that NK cells are cultured under appropriate culture conditions by using a bioreactor.

### [Technical Solution]

In an aspect, the present disclosure provides a method for producing NK cells, which includes:
(a) a step of stimulating a cell culture containing NK cells with feeder cells and then culturing the same stationarily;
(b) a step of suspension-culturing the stationarily cultured cell culture; and
(c) a step of re-stimulating the suspension-cultured cell culture by adding feeder cells at the time when the accumulated population doubling level of mononuclear cells in the cell culture reaches 3-5 and then suspension-culturing the same.

In another aspect, the present disclosure provides a method for producing NK cells, characterized in that NK cells are cultured under appropriate culture conditions by using an agitated bioreactor.

### [Brief Description of Drawings]

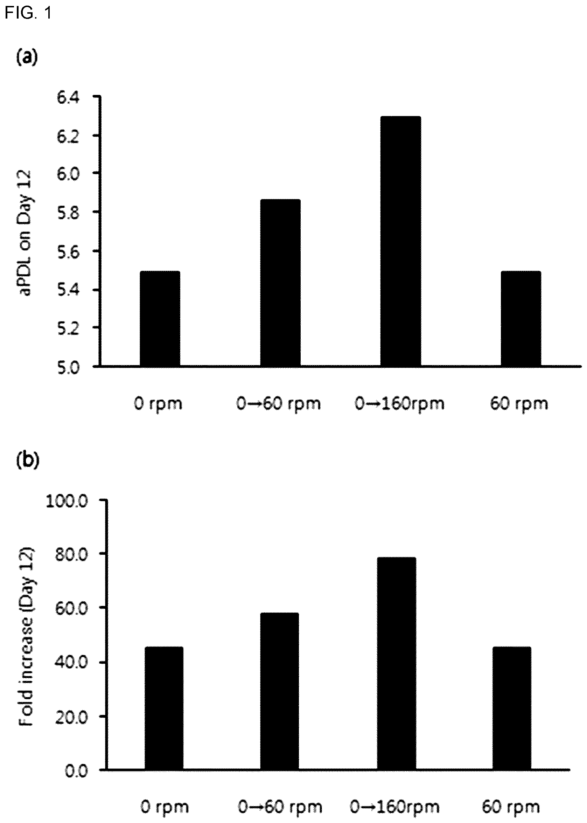

FIG. 1 shows the change in NK cell growth depending on stationary culture and suspension culture. 0 rpm: cells were cultured stationarily until day 12 day after thawing, 0→60 rpm: cells were cultured stationarily until day 7 after thawing and then suspension-cultured at agitation speed of 60 rpm until day 12, 0→160 rpm: cells were cultured stationarily until day 7 after thawing and then suspension-cultured at agitation speed of 160 rpm until day 12, 60 rpm: cells were suspension-cultured at agitation speed of 60 rpm until day 12 day after thawing.
(a) shows the growth of NK cells depending on stationary culture and suspension culture as accumulated population doubling level on day 12 after thawing.
(b) shows the growth of NK cells depending on stationary culture and suspension culture as fold increase on day 12 after thawing.

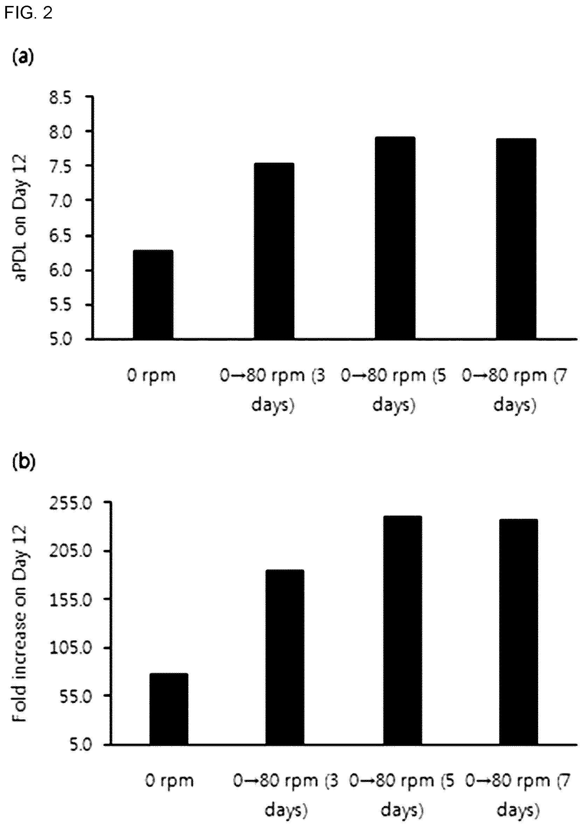

FIG. 2 shows the change in NK cell growth depending on the time of switching to suspension culture. 0 rpm: cells were cultured stationarily until day 12 day after thawing, 0→80 rpm (3 days): cells were cultured stationarily until day 3 after thawing and then suspension-cultured at agitation speed of 80 rpm until day 12, 0→80 rpm (5 days): cells were cultured stationarily until day 5 after thawing and then suspension-cultured at agitation speed of 80 rpm until day 12, 0→80 rpm (7 days): cells were cultured stationarily until day 7 after thawing and then suspension-cultured at agitation speed of 80 rpm until day 12.
(a) shows the growth of NK cells depending on the time of switching to suspension culture as accumulated population doubling level on day 12 after thawing.
(b) shows the growth of NK cells depending on the time of switching to suspension culture as fold increase on day 12 after thawing.

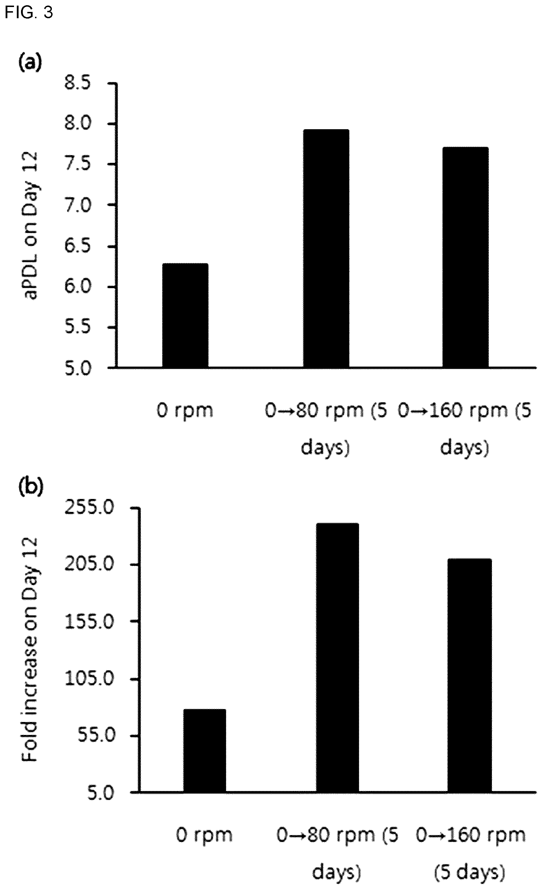

FIG. 3 shows the change in NK cell growth depending on agitation speed. 0 rpm: cells were cultured stationarily until day 12 day after thawing, 0→80 rpm (5 days): cells were cultured stationarily until day 5 after thawing and then suspension-cultured at agitation speed of 80 rpm until day 12, 0→160 rpm (5 days): cells were cultured stationarily until day 5 after thawing and then suspension-cultured at agitation speed of 160 rpm until day 12.
(a) shows the growth of NK cells depending on agitation speed as accumulated population doubling level on day 12 after thawing.
(b) shows the growth of NK cells depending on agitation speed as fold increase on day 12 after thawing.

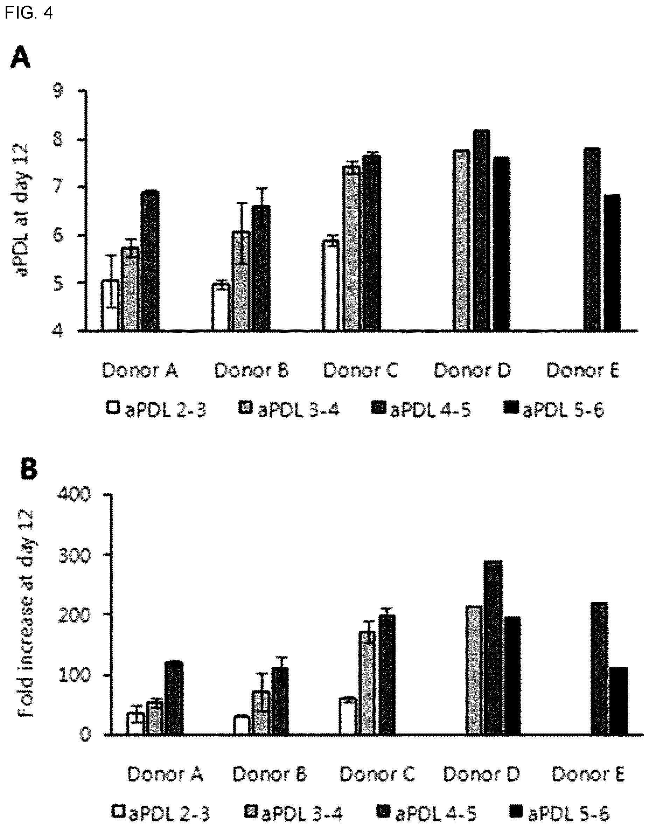

FIG. 4 shows the change in NK cell growth depending on the time of re-stimulation with feeder cells. aPDL 2-3: cells were re-stimulated with feeder cells when the accumulated population doubling level reached 2-3, aPDL 3-4: cells were re-stimulated with feeder cells when the accumulated population doubling level reached 3-4, aPDL 4-5: cells were re-stimulated with feeder cells when the accumulated population doubling level reached 4-5, aPDL 5-6 cells were re-stimulated with feeder cells when the accumulated population doubling level reached 5-6. The cells were acquired from a total of 5 donors.
(a) shows the growth of NK cells depending on the time of re-stimulation with feeder cells as accumulated population doubling level on day 12 after thawing.
(b) shows the growth of NK cells depending on the time of re-stimulation with feeder cells as fold increase on day 12 after thawing.

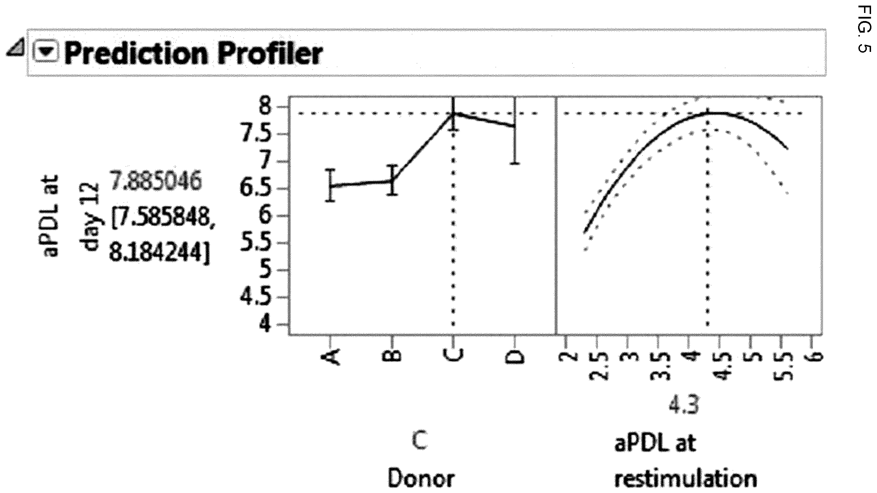

FIG. 5 shows a prediction profiler of accumulated population doubling level on day 12 after thawing depending on donors and the time of re-stimulation with feeder cells.

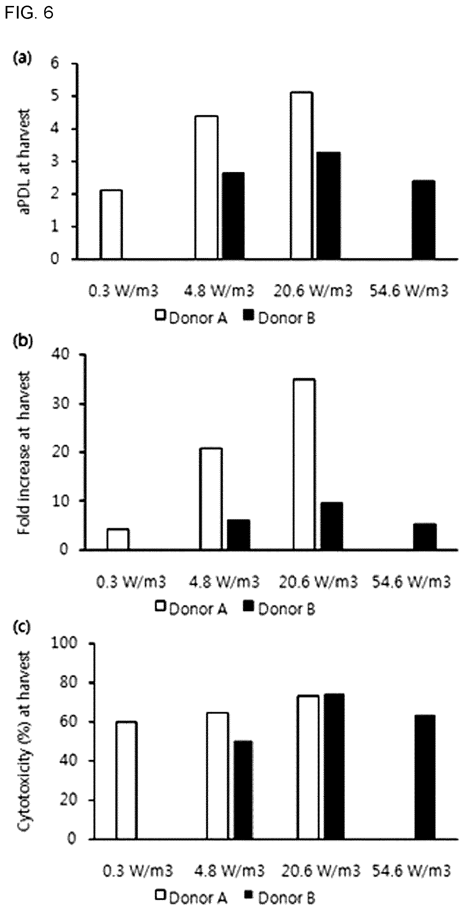

FIG. 6 shows the change in NK cell growth depending on the agitation speed of an agitated bioreactor. Agitation power per unit volume was 0.3 W/m³, 4.8 W/m³, 20.6 W/m³ or 54.6 W/m³ and cells acquired from a total of 2 donors were compared.
(a) shows the growth of NK cells depending on the agitation speed of an agitated bioreactor as accumulated population doubling level at the end of culturing.
(b) shows the growth of NK cells depending on the agitation speed of an agitated bioreactor as fold increase at the end of culturing.
(c) shows the growth of NK cells depending on the agitation speed of an agitated bioreactor as titer (cell-killing ability) at the end of culturing.

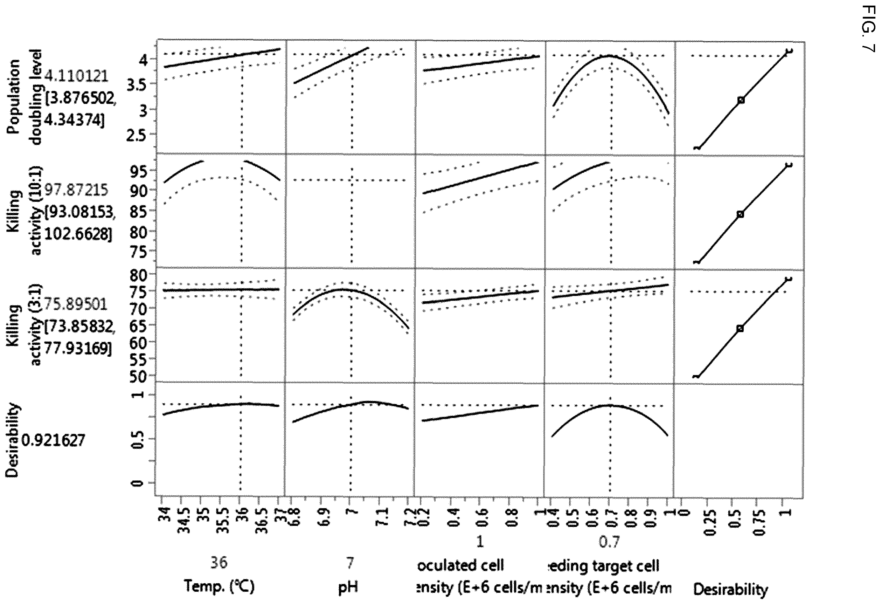

FIG. 7 shows prediction profilers of accumulated population doubling level, cell concentration at the end of culturing, titer (10:1) and titer (3:1) depending on the temperature of an agitated bioreactor, pH, inoculated cell concentration and target cell concentration.

### [Best Mode]

Unless defined otherwise, all the technical and scientific terms used herein have the same meaning as commonly understood by one of ordinary skill in the art to which the present disclosure belongs. It will be further understood that terms, such as those defined in commonly used dictionaries, should be interpreted as having a meaning that is consistent with their meaning in the context of the relevant art and the present disclosure.

The present disclosure provides a new method for producing NK cells for solving the problems of the existing method for producing high-purity NK cells that it is difficult to produce NK cells in large scale and that the killing ability of the produced NK cells is low.

In an aspect, the present disclosure provides a method for producing NK cells, which includes:
(a) a step of stimulating a cell culture containing NK cells with feeder cells and then culturing the same stationarily;
(b) a step of suspension-culturing the stationarily cultured cell culture; and
(c) a step of re-stimulating the suspension-cultured cell culture by adding feeder cells at the time when the accumulated population doubling level of mononuclear cells in the cell culture reaches 3-5 and then suspension-culturing the same.

In the present disclosure, (d) a step of obtaining the cultured NK cells may be further included after the step (c).

In the present disclosure, the suspension culturing of the step (c) may be performed for 1-30 days, specifically for 5-21 days, although not being limited thereto. In addition, the suspension culturing of the step (c) may be initiated 3-7 days, more specifically 4-6 days, most specifically 5 days, after the stimulation with feeder cells in the step (a).

Also, in the present disclosure, the re-stimulation with feeder cells in the step (c) may be started when the accumulated population doubling level (aPDL) reaches 2-6, specifically 3-5, more specifically 4-5, more specifically 4.1-4.6, most specifically 4.3, although not being limited thereto.

In the present disclosure, the suspension culturing in the step (c) may be performed at an agitation speed of 30-300 rpm, specifically 60-160 rpm. For example, it may be performed at an agitation speed of 70-90 rpm.

In the present disclosure, the feeder cells may be inactivated peripheral blood mononuclear cells, and a ratio of the feeder cells and mononuclear cells (seed cells) in the cell culture containing NK cells may be 5:1-1:15, specifically 2:1-1:10, more specifically 1:1-1:8, most specifically 1:4-1:6, although not being limited thereto.

In the present disclosure, the cell culture of the step (a) may be a culture containing peripheral blood mononuclear cells from which CD3-positive cells are removed. In this case, the peripheral blood mononuclear cells may be seed cells.

In the present disclosure, the culturing of the step (a) may be performed in a medium to which an anti-CD3 antibody is added, and the anti-CD3 antibody may be one or more selected from a group consisting of OKT3, UCHT1 and HIT3a. Also, in the present disclosure, the culturing of the step (a) may be performed in a medium to which IL-2 is added.

In the present disclosure, stationary culture refers to culturing in a reactor without agitation or shaking, and suspension culture refers to culturing cells in a suspended state without being attached to the bottom or side surface of a reactor through aeration, agitation, etc.

The reactor that may be used for stationary culture in the present disclosure may be a shaking flask, a T-flask, a disposable cell culture bag, etc., although not being limited thereto. And, the reactor that may be used for suspension culture in the present disclosure may be a shaking flask, a shaking reactor, a T-flask, a disposable cell culture bag, etc., although not being limited thereto. However, any reactor that can be easily selected by one of ordinary skill in the art to which the present disclosure belongs may be used as a bioreactor suitable to accomplish the purpose of the present disclosure.

The reactor for stationary culture and the reactor for suspension culture may be identical to or different from each other. For example, when the reactor for stationary culture and the reactor for suspension culture are identical, after stationary culture is completed, suspension culture may be performed in the same reactor by additionally supplying a medium containing necessary nutrients such as cytokines, etc. And, when different types of reactors are used, after stationary culture is completed, suspension culture may be performed after transferring the culture to a reactor for suspension culture.

Examples of the reactor for suspension culture include GE Healthcare's Wave Bioreactor, ThermoFisher's Single-Use Bioreactor (SUB), Xcellerex's Single-Use XDR Bioreactor, Sartorius's Biostat STR®, Nipro's cell culture bag, PBS Biotech's PBS series bioreactors (PBS Mini, PBS3, PBS15, PBS80, PBS500, etc.; see WO 07/111677A, WO08/133845A, WO 09/132192A, etc.), Fujimori Kogyo's cell culture bag, Erlenmeyer's Disposable Shake Flasks, etc., although not being limited thereto. For example, PBS Biotech's PBS series bioreactors may be used.

In the present disclosure, the 'feeder cells' refer to cells which lack the ability to divide but have metabolic activity and thus help the proliferation of target NK cells by producing several metabolites. The feeder cells that can be used in the present disclosure include gene-introduced animal cells, peripheral blood leukocytes (PBL) treated with various cytokines or chemicals, autologous or allogeneic peripheral blood leukocytes (PBL), T cells, B cells, monocytes, etc., specifically autologous peripheral blood mononuclear cells, although not being limited thereto. It is obvious that other feeder cells commonly known in the art to which the present disclosure belongs can be used without limitation as long as they are congruent with the purpose of the present disclosure.

The autologous peripheral blood mononuclear cells used as feeder cells may be inactivated to ensure safety. For inactivation, common methods known in the art may be used. For example, gamma-ray irradiation may be used. The inactivated feeder cells include isolated T cells. The method of proliferating NK cells together with feeder cells according to the present disclosure is advantageous in that, since NK cells are isolated purely and then proliferated, only pure NK cells proliferate thereafter.

In the present disclosure, the anti-CD3 antibody is an antibody binding specifically to a CD3 antigen, which is a molecule that binds to the T-cell receptor (TCR) and forms an antigen-recognizing complex. The CD3 molecule serves to bind to the TCR and transports an antigen recognition signal in the cell.

The anti-CD3 antibody that can be used in the present disclosure may be any antibody binding to CD3 without limitation. For example, one selected from a group consisting of OKT3, UCHT1 and HIT3a may be used, although not being limited thereto.

In the present disclosure, the cytokine that may be contained in the medium may be one or more selected from interleukins. Interleukins collectively refer to protein-based biologically active substances produced by immune cells such as lymphocytes, mononuclear cells, macrophages, etc. The interleukins that can be used in the present disclosure may be one or more selected from a group consisting of interleukin-2 (IL-2), interleukin-12 (IL-12), interleukin-15 (IL-15), interleukin-18 (IL-18) and interleukin-21 (IL-21), particularly IL-2, although not being limited thereto. However, it is obvious to those of ordinary skill in the art to which the present disclosure that other cytokines can also be used without limitation as long as they are congruent with the purpose of the present disclosure.

The concentration of the anti-CD3 antibody in the medium used for stationary culture and suspension culture in the present disclosure may be 0.1-1,000 ng/mL, specifically 1-100 ng/mL, more specifically 5-20 ng/mL, and the concentration of the cytokine in the medium may be 10-2,000 IU, specifically 100-1,000 IU, more specifically about 200-700 IU.

In the present disclosure, 'stimulation' refers to inducing the proliferation of NK cells by adding feeder cells, etc. and an anti-CD3 antibody may be used together.

In the present disclosure, 're-stimulation' refers to, after a lapse of a certain culture period, re-inducing the proliferation of NK cells by adding feeder cells and/or an anti-CD3 antibody to the medium.

As the medium for producing NK cells according to the present disclosure, a common medium for animal cell culture such as a CELLGRO medium (Cellgenix), an AIM-V medium, a RPMI1640 medium, XVIVO 20, etc. may be used. If necessary, the medium for animal cell culture may be supplemented with one or more components selected from NK cells isolated from human peripheral blood, peripheral blood mononuclear cells, anti-CD3 antibodies and interleukins.

In particular, in the method for producing NK cells of the present disclosure, in order to maintain the concentrations of cells and cytokines constant during suspension culture, the concentrations of cells and cytokines may be measured at predetermined time intervals and the medium containing cytokines may be provided according to the measurement result.

In addition, the medium may contain serum or plasma and additional proliferation factors that support the proliferation of lymphocytes.

The serum or plasma added to the medium is not specially limited and commercially available ones derived from various animals may be used. Specifically, human-derived autologous serum or plasma may be used. For example, methods known to those of ordinary skill such as addition of a combination of cytokines proliferating lymphocytes from peripheral blood mononuclear cells, lectins stimulating the proliferation of lymphocytes, etc. may be used.

The NK cells prepared by the method according to the present disclosure may be provided as a therapeutic composition together with an adequate excipient and an additive. The composition may be administered to a patient in need of treatment to achieve a therapeutic effect.

Also, since the NK cells are produced with the concentration of the cells maintained, cell overgrowth can be prevented and the optimum cell state can be maintained. In particular, the function of the cells can be retained and high cell survival rate and cell-killing ability can be maintained even when they are frozen and then thawed. Accordingly, the cells can be stored and supplied in liquid or frozen state without a further treatment process.

The NK cells produced by the method according to the present disclosure and a composition containing the same may be used for treatment of tumors and infectious diseases. The NK cells produced by the method according to the present disclosure is applicable to all types of tumors including solid cancer and blood cancer. Solid cancer refers to a cancer formed as a lump in an organ, unlike blood cancer, and includes cancers occurring in most organs. The tumor that can be treated using the NK cells according to the present disclosure is not specially limited and includes stomach cancer, liver cancer, lung cancer, colon cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, lymphoma, etc., although not being limited thereto. In the present disclosure, the infectious disease refers to a disease caused by infection with viruses or pathogens and includes all diseases that can be infected through respiratory organs, blood, skin contact, etc. Non-limiting examples of the infectious disease include hepatitis B and C, human papilloma virus (HPV) infection, cytomegalovirus infection, viral respiratory diseases, influenza, etc., although not being limited thereto.

In another aspect, the present disclosure relates to a method for producing NK cells, which includes a step of inoculating a cell culture including NK cells to a bioreactor.

In the present disclosure, a "bioreactor" refers to a culture apparatus allowing continuous control of conditions that affect cell growth such as dissolved oxygen concentration, dissolved carbon dioxide concentration, pH, temperature, etc.

Specifically, the bioreactor that can be used in the present disclosure may be an agitated bioreactor. For instance, Storius's BIOSTAT may be used as the bioreactor, although not being limited thereto. In the present disclosure, the "agitated bioreactor" refers to a bioreactor which allows, in addition to the continuous control of culture conditions described above, adequate agitation, i.e. control of agitation power. Specifically, it may be a bioreactor which also allows continuous supply of a medium. More specifically, the bioreactor that can be used in the present disclosure may be a bioreactor whereby agitation of a culture medium can be achieved through rotation of an impeller, etc. of the reactor, although not being limited thereto.

Use of the agitated bioreactor allows the control of appropriate culture conditions, e.g., adequate or optimum dissolved oxygen concentration, dissolved carbon dioxide concentration and pH of the culture medium. Also, when NK cells are produced in the culture temperature range at a specific agitation power per unit volume using the bioreactor, the quantity of the NK cells obtained and their killing ability are improved remarkably.

In the method for producing NK cells using an agitated bioreactor, the pH of the culture condition may be 6.5-7.6, specifically 6.8-7.2, specifically 6.9-7.1, most specifically 7.0-7.07, and the culture temperature may be 25-40 °C, specifically 33-40 °C, more specifically 34-38 °C, most specifically 35-37.5 °C, although not being limited thereto.

In addition, the agitation power per unit volume may be 0.1-100 W/m³, specifically 0.3-80 W/m³, more specifically 5-60 W/m³, most specifically 10-30 W/m³, although not being limited thereto.

The method for producing NK cells using the bioreactor may be carried out by inoculating a cell culture obtained by performing stationary culture and suspension culture sequentially to a bioreactor and culturing the same.

The concentration of the NK cells in the bioreactor immediately after the inoculation, i.e., the concentration of the NK cells in the bioreactor at the time of inoculation, may be 0.1-2.0x10⁶ cells/mL, specifically 0.2-1.5x10⁶ cells/mL, more specifically 0.8-1.2x10⁶ cells/mL, most specifically 0.9-1.1x10⁶ cells/mL, although not being limited thereto.

During the culture period, a target cell concentration (cell concentration after addition of additives, or feeding target cell density) may be 0.4-1.0x10⁶ cells/mL, specifically 0.55-0.85x10⁶ cells/mL, more specifically 0.6-0.8x10⁶ cells/mL, most specifically 0.65-0.75x10⁶ cells/mL, although not being limited thereto.

During the culture of NK cells in the bioreactor, as additives such as an additional medium, etc. is added to the culture medium and the volume of the culture medium is increased, the NK cells are diluted and the concentration thereof is decreased. The target cell concentration refers to the concentration of NK cells in the culture medium after addition of the additives such as a culture medium, etc.

Specifically, the cell culture used for the inoculation of the bioreactor may be a culture of NK cells produced by the method for producing NK cells of the present disclosure, although not being limited thereto.

### [Mode for Invention]

Hereinafter, the present disclosure will be described in detail through examples. However, the following examples are for illustrative purposes only and it will be obvious to those of ordinary skill in the art that the scope of the present disclosure should not be interpreted to be limited by the examples.

### Example 1. Investigation of NK cell growth by suspension culture

### (1) Culture of NK cells

In order to remove CD3-positive cells from peripheral blood mononuclear cells (PBMC) collected from healthy donors and obtain feeder cells (PBMC), the cells were transferred to a fresh 50-mL tube, with 5x10⁷ cells per each. Then, the cells were centrifuged at 1,200 rpm and 4 °C for 10 minutes. After the centrifugation, the recovered feeder cells (1x10⁸ cells) were dispensed into a vial and frozen in a liquid nitrogen tank. The frozen cells were thawed before use.

In order to obtain PBMCs from which CD3-positive cells are removed, 400 µL of a MACS running buffer (Miltenyi Biotech, Korea) and 100 µL of CD3 magnetic beads (Miltenyi Biotech, Korea) were added to cell pellets consisting of 5x10⁷ cells and were allowed to react at 4 °C for 20 minutes. Then, the cells were with 20 mL of a MACS running buffer, centrifuged at 1,200 rpm and 4 °C for 10 minutes and then suspended again in 2 mL of a MACS running buffer. The cells were separated from the suspension by loading in a CS column (Miltenyi Biotech, 130-041-305) equipped with VarioMACS (Miltenyi Biotech, Korea), and the column was washed to reach a final volume of 20 mL, thereby recovering PBMC cells with CD3-positive cells removed. 2x10⁷ of the recovered cells were dispensed into a vial and frozen in a liquid nitrogen tank. The frozen cells were thawed before use.

After thawing the frozen PBMCs with CD3-positive cells in a water bath at 37 °C, the number of the cells was counted. About 2x10⁷ cells were transferred to a fresh 50-mL tube and centrifuged at 1,200 rpm and 4 °C for 10 minutes. Cell pellets obtained through centrifugation were suspended in 10 mL of a CELLGRO medium (Cellgenix, USA) containing 10 mL of 2 vol% plasma.

After thawing the frozen feeder cells in a 37 °C water bath at 37 °C, the number of the cells was counted. About 1x10⁸ cells were transferred to a fresh 50-mL tube and centrifuged at 1,200 rpm and 4 °C for 10 minutes. Cell pellets were suspended in 10 mL of a CELLGRO medium (Cellgenix, USA) containing 2 vol% plasma and the feeder cells were inactivated by irradiating with about 2000 cGy using a gamma-ray irradiator.

For co-culture of the inactivated feeder cells and the PBMCs from which CD3-positive cells were removed, after adding 500-1,000 IU of IL-2 and 10 ng/mL of OKT-3 to the tube holding the feeder cells, a CELLGRO medium (Cellgenix, USA) containing 20 mL of the feeder cells, 20 mL of the PBMCs from which CD3-positive cells were removed and 20 mL of 1 vol% plasma was added to a shake flask and the cells were cultured in a 37 °C reactor for 5 days.

After diluting the cells by adding 60 mL of a CELLGRO medium (Cellgenix, USA) containing 500-1,000 IU of IL-2 and 1 vol% plasma to the shake flask, the cells were cultured in a suitable culture reactor.

On day 7 after the beginning of the culture, the number of cells was counted. The cells were diluted with a CELLGRO medium containing 1 vol% plasma to a cell concentration of 2x10⁵-5x10⁵ cells/mL. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After culturing for 3 days, the cells were counted, diluted to 5-10x10⁵ cells/mL with a CELLGRO medium containing 500-1,000 IU of IL-2 and 1 vol% plasma, and then cultured for 2 days.

### (2) Investigation of NK cell growth by stationary culture and suspension culture

In order to investigate the difference in NK cell growth by stationary culture and suspension culture, NK cells were grown in a reactor under the condition of 37 °C and 5% CO₂. The cells were cultured under the stationary culture and suspension culture conditions described in Table 1.

For condition #1, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then cultured stationarily in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After culturing stationarily for 3 days, the cells were counted, diluted to 5-10x10⁵ cells/mL with a CELLGRO medium containing 500-1,000 IU of IL-2 and 1 vol% plasma, and then cultured stationarily for 2 days.

For condition #2 and condition #3, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then cultured stationarily in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation. For the condition #2, suspension culture was conducted at an agitation speed of 60 rpm, and, for the condition #3, suspension culture was conducted at an agitation speed of 160 rpm.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

For condition #4, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, suspension culture was conducted in a 37 °C reactor for 5 days at an agitation speed of 60 rpm by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then suspension-cultured in an adequate culture reactor. On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

The total culture period of the conditions #1-4 was 12 days.

The growth of the cells that had been cultured for 12 days is compared in FIG. 1 as accumulated population doubling level (aPDL) and fold increase. The accumulated population doubling level (aPDL) of cells means the number of cell divisions after thawing, and the fold increase means what times the cells grew after the thawing. The cells that had been cultured stationarily for 7 days and then suspension-cultured (conditions #2 and #3) showed better cell growth as compared to those cultured stationarily for 12 days (condition #1). However, the cells that were suspension-cultured since immediately after thawing (condition #4) did not show good results. The cells that were suspension-cultured after stationary culture for 7 days showed difference in cell growth depending on the agitation speed. The highest cell growth was achieved at 160 rpm (condition #3).

**[Table 1]**

| Time of beginning of suspension culture and agitation speed | | | | |
|---|---|---|---|---|
| Condition # | Reactor | Culture environment | Beginning of suspension culture | Agitation speed (rpm) |
| 1 | Shake flask | Stationary culture | - | 0 |
| 2 | Shake flask | Stationary culture → suspension culture | Day 7 after thawing | 60 |
| 3 | Shake flask | Stationary culture → suspension culture | Day 7 after thawing | 160 |
| 4 | Shake flask | Suspension culture | Immediately after thawing | 60 |

### (3) Investigation of NK cell growth depending on time of conversion to suspension culture

In order to investigate whether there is a difference in NK cell growth depending on the time of conversion to suspension culture, NK cells were cultured in an incubator under the condition of 37 °C and 5% CO₂.

Culture conditions (conditions #1-4) about the time of conversion to suspension culture are summarized in Table 2. After thawed cells were stationarily for 3 days, 5 days or 7 days, and then suspension-cultured. The suspension culture was performed at an agitation speed of 80 rpm.

The culturing was performed as follows.

For condition #1 (control group), suspension culture was not conducted. After thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then cultured stationarily in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

For condition #2, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 3 days by adding 20 mL of feeder cells, 20 mL of PBMCs from which CD3-positive cells were removed and 20 mL of a CELLGRO medium containing 1 vol% plasma to a shake flask.

After culturing for 3 days, the cells were diluted by adding 60 mL of a CELLGRO medium containing 500-1,000 IU of IL-2 and 1 vol% plasma to the shake flask and then suspension-cultured for 4 days at an agitation speed of 80 rpm in an adequate culture reactor.

Then, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

For condition #3, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then suspension-cultured for 2 days at an agitation speed of 80 rpm in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation. After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

For condition #4, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then cultured stationarily in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation. The cells were suspension-cultured at an agitation speed of 80 rpm.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

After conducting culturing for a total of 12 days under the conditions #1-4, accumulated population doubling level and fold increase were compared.

As shown in FIG. 2, the cells that were suspension-cultured since days 3, 5 and 7 after thawing showed superior cell growth as compared to the stationarily cultured cells. The highest cell growth was achieved 5 days after the stimulation with feeder cells, but there was no significant difference from 3-7 days after the stimulation.

**[Table 2]**

| Time of beginning of suspension culture | | | | |
|---|---|---|---|---|
| Condition # | Reactor | Culture environment | Beginning of suspension culture | Agitation speed (rpm) |
| 1 | Shake flask | Stationary culture | - | 0 |
| 2 | Shake flask | Stationary culture → suspension culture | Day 3 after thawing | 80 |
| 3 | Shake flask | Stationary culture → suspension culture | Day 5 after thawing | 80 |
| 4 | Shake flask | Stationary culture → suspension culture | Day 7 after thawing | 80 |

### (4) Investigation of NK cell growth depending on agitation speed

In order to investigate NK cell growth depending on agitation speed during suspension culture, NK cells were cultured in an incubator under the condition of 37 °C and 5% CO₂.

Culture conditions about the agitation speed are summarized in Table 3.

For condition #1 (control group), suspension culture was not conducted. After thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then cultured stationarily in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation.

After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

For condition #2 and condition #3, after thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then suspension-cultured for 2 days in an adequate culture reactor. The suspension culture was performed at an agitation speed of 80 rpm for the condition #2, and the suspension culture was performed at an agitation speed of 160 rpm for the condition #3.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation. After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

After conducting culturing for a total of 12 days, accumulated population doubling level and fold increase were compared. The result is shown in FIG. 3.

Taken together with the result of Example 1(2), the cells suspension-cultured at an agitation speed of 60-160 rpm showed higher cell growth as compared to the stationarily cultured cells. No significant change in cell growth was observed between 80 and 160 rpm.

**[Table 3]**

| Agitation speed of shaker during suspension culture | | | | |
|---|---|---|---|---|
| Condition # | Reactor | Culture environment | Beginning of suspension culture | Agitation speed (rpm) |
| 1 | Shake flask | Stationary culture | - | 0 |
| 2 | Shake flask | Stationary culture → suspension culture | Day 5 after thawing | 80 |
| 3 | Shake flask | Stationary culture → suspension culture | Day 5 after thawing | 160 |

### Example 2. Investigation of NK cell growth depending on time of repeated stimulation (re-stimulation) using feeder cells

In order to investigate the time of beginning of re-stimulation with feeder cells during NK cell culture, re-stimulation with feeder cells was conducted at the time of reaching a specific accumulated population doubling level. Culturing was conducted in a reactor under the condition of 37 °C and 5% CO₂ as in Example 1(1), except that the re-stimulation with feeder cells was conducted at the time of reaching accumulated population doubling level (aPDL) of 2-3, 3-4, 4-5 and 5-6, respectively, instead of day 7 after the beginning of the culturing (Table 4).

The culturing was conducted as follows.

After thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then suspension-cultured for 2 days in an adequate culture reactor.

After counting the cells, when the accumulated population doubling level (aPDL) reached 2-3, 3-4, 4-5 and 5-6, respectively, the cells were diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured and the feeder cells prepared for re-stimulation) were co-cultured additionally for 3 days for re-stimulation. After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days. Then, the accumulated population doubling level and fold increase were compared (Table 4).

The accumulated population doubling level is a measure of the number of cell divisions after thawing, and the fold increase means what times the cells grew after the thawing.

As shown in FIG. 4, all donors (donors A to E) showed the highest cell growth when re-stimulated with feeder cells at aPDL 4-5. When re-stimulated with feeder cells at aPDL 5-6, the cell growth was decreased as compared to when re-stimulated with feeder cells at aPDL 4-5.

A regression model was derived from donors, time of re-stimulation with feeder cells (based on accumulated population doubling level) and square term of re-stimulation with feeder cells (based on accumulated population doubling level) as input values. The output value of the regression model was the accumulated population doubling level on day 12 after culturing, and JMP13 was used as a statistical program for deriving the regression model. The R² and R²_{adj} values of the regression model were 0.90 and 0.88, respectively, and the P value statistically significant with 0.001 or smaller. The P values for the time of re-stimulation with feeder cells and the square term of the time of re-stimulation with feeder cells were also statistically significant with 0.001 or smaller. A profiler was obtained using the prediction model as shown in FIG. 5. The time of re-stimulation with feeder cells showed a parabolic curve with a vertex around 4.3, suggesting that there exists an optimum time of re-stimulation with feeder cells based on the accumulated population doubling level.

**[Table 4]**

| Time of repeated stimulation (re-stimulation) using feeder cells | | |
|---|---|---|
| Condition # | Donor | Time of re-stimulation (aPDL) |
| 1 | Donor A | 2-3 |
| 2 | | 3-4 |
| 3 | | 4-5 |
| 4 | Donor B | 2-3 |
| 5 | | 3-4 |
| 6 | | 4-5 |
| 7 | Donor C | 2-3 |
| 8 | | 3-4 |
| 9 | | 4-5 |
| 10 | Donor D | 3-4 |
| 11 | | 4-5 |
| 12 | | 5-6 |
| 13 | Donor E | 4-5 |
| 14 | | 5-6 |

### Example 3. Development and characterization of agitated bioreactor process

In this example, a bioreactor process capable of culturing NK cells in large quantities under a uniform environment was developed and its characteristics were analyzed.

### (1) Investigation of NK cell growth depending on agitation speed of agitated bioreactor

NK cells cultured for about 12 days were inoculated to an agitated bioreactor and then cultured for up to 21 day by a fed-batch culture method.

The culturing was performed as follows.

Frozen feeder cells and frozen PBMCs from which CD3-positive cells were removed were thawed and then cultured stationarily for 5 days. Then, the cell culture was converted to suspension culture (agitation speed 160 rpm). The cells were cultured for a total of 12 days.

After thawing frozen feeder cells and frozen PBMCs from which CD3-positive cells were removed and inactivating the feeder cells by irradiating with about 2000 cGy using a gamma-ray irradiator, 500-1,000 IU of IL-2 and 10 ng/mL of OKT-3 were added to a tube holding the feeder cells in order to co-culture the inactivated feeder cells and the PBMCs from which CD3-positive cells were removed. Then, stationary culture was conducted in a 37 °C reactor for 5 days by adding 20 mL of feeder cells, 20 mL of PBMCs from which CD3-positive cells were removed and 20 mL of a CELLGRO medium containing 1 vol% plasma to a shake flask.

After culturing for 5 days, the cells were diluted by adding 60 mL of a CELLGRO medium containing 500-1,000 IU of IL-2 and 1 vol% plasma to the shake flask and then suspension-cultured for 2 days at an agitation speed of 160 rpm in an adequate culture reactor.

On day 7 after the beginning of the culture, the cells were counted and diluted to a cell concentration of 2x10⁵-5x10⁵ cells/mL with a CELLGRO medium containing 1 vol% plasma. For re-stimulation with feeder cells, 5-fold feeder cells were prepared and suspended in a CELLGRO medium containing 1 vol% plasma. After inactivating the feeder cells by irradiating with about 2000 cGy using a gamma-ray irradiator and adding 500-1,000 IU of IL-2 and 10 ng/mL of OKT-3, the prepared two cells (the cells that had been co-cultured for 7 days and the feeder cells prepared for re-stimulation) were co-cultured additionally for 3 days for re-stimulation. After suspension-culturing for 3 days, the cells were counted, diluted to 5-10x10⁵ cells/mL with a CELLGRO medium containing 500-1,000 IU of IL-2 and 1 vol% plasma, and then suspension-cultured for 2 days.

The NK cells that had been cultured for 12 days were inoculated to an agitated bioreactor and then fed-batch cultured for up to 21 days.

The agitated bioreactor was set to a culturing temperature of 37 °C, pH 7.05 and DO 50%, and the inoculated cell concentration was 0.5x10⁶ cells/mL. After the inoculation, a medium was added to a cell concentration of 1.0x10⁶ cells/mL at 1- to 3-day intervals. The agitation speed of the bioreactor was set to 0.3 W/m³, 4.8 W/m³, 20.6 W/m³ and 54.6 W/m³ based on power per volume (P/V). After culturing for 8 days, the culturing was terminated, and accumulated population doubling level and fold increase were compared as shown in FIG. 6. The cell growth showed significant difference depending on the agitation speed, with the highest growth at 20.6 W/m³. In addition, the titer (cell-killing ability) of the cultured NK cells measured according to the method of (2) at the end of culturing. The highest titer was observed at 20.6 W/m³.

### (2) Measurement of in-vitro cell-killing ability

After recovering target tumor cells (K562, ATCC CCL-243), 3x10⁶ cells were transferred to a 15-mL tube and centrifuged at 1,200 rpm and 4 °C for 5 minutes. Cell pellets obtained through the centrifugation were suspended in a RPMI medium to a concentration of 1x10⁶ cells/mL and then 1 mL of tumor cells were transferred to a fresh 15-mL tube. After adding 30 µL of 1 mM calcein-AM (Molecular Probes, C34852), the 15-mL tube was covered with aluminum foil and stained in a 37 °C incubator for 1 hour. After washing the tumor cells stained with calcein-AM by adding 10 mL of a RPMI medium, followed by centrifugation at 1,200 rpm and 4 °C for 5 minutes, cell pellets were suspended in 10 mL of a RPMI medium to a cell concentration of 1x10⁵ cells/mL.

After thawing frozen NK cells and transferring to a 15-mL tube, followed by centrifugation at 1,200 rpm and 4 °C for 10 minutes, cell pellets were suspended in a RPMI medium with a desired ratio with respect to target tumor cells. 100 µL of the prepared target tumor cells and NK cells were mixed and plated on a roundbottomed 96-well plate. Each well was prepared in triplicates and average was taken. After reacting the plate in a 37 °C incubator for 4 hours with light blocked, the plate was centrifuged at 2,000 rpm and 4 °C for 3 minutes. After transferring 100 µL of the culture in each well to a 96-well black plate, fluorescence was measured under the condition of excitation (485 nm)/emission (535 nm) for 0.1 second using a fluorescence spectrometer.

### (3) Optimization of agitated bioreactor process

For optimization of an agitated bioreactor process, NK cells were cultured for about 12 days according to the method described in Example 3(1). The NK cells cultured for 12 days were inoculated to an agitated bioreactor and then cultured for up to 22 days by the fed-batch culture method.

The culture condition used in this example is described in Table 5. DOE analysis (definitive screening designs, 9 conditions, Table 6) was carried out using four parameters, temperature, pH, inoculated cell concentration and target cell concentration after addition of additives, which affect cell growth and titer (cell-killing ability, Example 3(2)) of NK cells. During the culture period, additives were added with 1- to 3-day intervals and culturing was completed after 8-10 days.

**[Table 5]**

| Parameters of bioreactor process | | | |
|---|---|---|---|
| Parameter | Minimum | Intermediate | Maximum |
| Agitation speed (rpm) | 260 | | |
| DO (%) | 50 | | |
| Temperature (°C) | 34 | 35.5 | 37 |
| pH | 6.8 | 7.0 | 7.2 |
| Inoculated cell concentration (x10⁶ cells/mL) | 0.2 | 0.6 | 1.0 |
| Target cell concentration (x10⁶ cells/mL) | 0.4 | 0.7 | 1.0 |

**[Table 6]**

| DOE experiment condition (DSP, definitive screening designs) | | | | |
|---|---|---|---|---|
| Condition # | Inoculated cell concentration (x10⁶ cells/mL) | Target cell concentration (x10⁶ cells/mL) | Temperature (°C) | pH |
| 1 | 0.6 | 1.0 | 37 | 7.2 |
| 2 | 0.6 | 0.4 | 34 | 6.8 |
| 3 | 1.0 | 0.7 | 37 | 6.8 |
| 4 | 0.2 | 0.7 | 34 | 7.2 |
| 5 | 1.0 | 0.4 | 35.5 | 7.2 |
| 6 | 0.2 | 1.0 | 35.5 | 6.8 |
| 7 | 1.0 | 1.0 | 34 | 7.0 |
| 8 | 0.2 | 0.4 | 37 | 7.0 |
| 9 | 0.6 | 0.7 | 35.5 | 7.0 |

After the experiment was completed, aPDL and cell concentration and titer (10:1 and 3:1) at the time of harvest were measured and analyzed with JMP11 (P-value threshold: P ≥ 0.25). The R² and R²_{adj} values were 0.90 or higher and 0.80 or higher, respectively, and the P value statistically significant with 0.05 or smaller.

The R² analysis result is shown in Table 7. A P-value of 0.05 or smaller means that the parameter affects the result (accumulated population doubling level and titer) statically significantly. It was found out that all the four process parameters (temperature, pH, inoculated cell concentration and target cell concentration) significantly affect the result. Prediction profilers were created based on the analysis result, as shown in FIG. 7, and the optimal points of the bioreactor process parameters were identified. The culture conditions for maintaining all of the accumulated population doubling level, titer (10:1) and titer (3:1) the highest are: inoculated cell density 1.0x10⁶ cells/mL, target cell concentration 0.7x10⁶ cells/mL, temperature 36 °C and pH 7.

**[Table 7]**

| Significance of bioreactor process parameters for growth and titer | | | |
|---|---|---|---|
| Parameter | Accumulated population doubling level | Titer (10:1, %) | Titer (3:1, %) |
| R² | 0.982 | 0.901 | 0.995 |
| R²adj | 0.950 | 0.819 | 0.987 |
| P value | 0.003 | 0.006 | ≥ 0.001 |
| Lack of fit (P value) | 0.339 | 0.981 | 0.275 |

Although particular aspects of the present disclosure have been described in detail above, it will be apparent to those of ordinary skill in the art that such specific description is merely about specific exemplary embodiments and the scope of the present disclosure is not limited by them. It is to be noted that the substantial scope of the present disclosure will be defined by the appended claims and their equivalents.

### [Industrial Applicability]

The production method according to the present disclosure has an advantage that NK cells having a high cell-killing ability and cell survival rate can be produced with high purity and at high efficiency in a short period of time by a clinically friendly method as compared with existing methods, thereby increasing the productivity of an NK cell therapy agent.

Although particular aspects of the present disclosure have been described in detail above, it will be apparent to those of ordinary skill in the art that such specific description is merely about specific exemplary embodiments and the scope of the present disclosure is not limited by them. It is to be noted that the substantial scope of the present disclosure will be defined by the appended claims and their equivalents.

## Claims

1. A method for producing NK cells, comprising:
(a) stimulating a cell culture comprising NK cells with feeder cells and then culturing the same stationarily;
(b) suspension-culturing the stationarily cultured cell culture; and
(c) re-stimulating the suspension-cultured cell culture by adding feeder cells at the time when the accumulated population doubling level of mononuclear cells in the cell culture reaches 3-5 and then suspension-culturing the same.

2. The method for producing NK cells according to claim 1, wherein the suspension culturing of the step (b) is initiated 3-7 days after the stimulation with feeder cells in the step (a).

3. The method for producing NK cells according to claim 1, wherein the suspension culturing of the step (c) is performed at an agitation speed of 30-300 rpm.

4. The method for producing NK cells according to claim 1, wherein the culturing of the step (a) is performed in a medium to which an anti-CD3 antibody is added.

5. The method for producing NK cells according to claim 4, wherein the anti-CD3 antibody is one or more selected from a group consisting of OKT3, UCHT1 and HIT3a.

6. The method for producing NK cells according to claim 1, wherein the culturing of the step (a) is performed in a medium to which one or more cytokine selected from a group consisting of interleukin-2 (IL-2), interleukin-12 (IL-12), interleukin-15 (IL-15), interleukin-18 (IL-18) and interleukin-21 (IL-21) is added.

7. The method for producing NK cells according to claim 1, wherein the feeder cells are inactivated peripheral blood mononuclear cells.

8. The method for producing NK cells according to claim 1, wherein the cell culture of the step (a) comprises peripheral blood mononuclear cells from which CD3-positive cells are removed.

9. A method for producing NK cells, comprising a step of inoculating a cell culture comprising NK cells to a bioreactor.

10. The method for producing NK cells according to claim 9, wherein the bioreactor is controlled to pH 6.5-7.6, culture temperature of 25-40 °C and agitation power per unit volume of 0.1-100 W/m³.

11. The method for producing NK cells according to claim 9, wherein the cell culture is subjected to stationary culture and suspension culture sequentially.

12. The method for producing NK cells according to claim 9, wherein the concentration of NK cells in the bioreactor at the time of the inoculation is 0.1-2.0x10⁶ cells/mL.

13. The method for producing NK cells according to claim 9, wherein a target cell concentration (cell concentration after addition of additives, or feeding target cell density) during the culture period is 0.4-1.0x10⁶ cells/mL.

14. The method for producing NK cells according to claim 9, wherein the cell culture is a culture of the NK cells produced by the method according to any of claims 1 to 8.

15. NK cells produced by the method according to any of claims 1 to 8.

16. NK cells produced by the method according to any of claims 9 to 14.
